# EUROPEAN PATENT APPLICATION

(11) **EP 4 548 965 A1**
(43) Date of publication of application: **07.05.2025**
(21) Application number: 24208767.4
(22) Date of filing: 24.10.2024
(51) Int. Cl.: A61N 1/375, H01G 4/35

(54) **SINGLE-SIDED OR SINGLE-ENDED SYSTEM GROUND FOR A RECTANGULAR OR SQUARE AIMD EMI FILTER CAPACITOR**

(30) Priority: 02.11.2023 US 202363595511 P; 21.10.2024 US 202418921348
(71) Applicant: Greatbatch Ltd., Clarence, New York 14031 (US)
(72) Inventor: STEVENSON, Robert, Canyon Country 91387 (US)
(74) Representative: Mathys & Squire

(57) **Abstract**

A filtered feedthrough comprises an insulator sealed in a ferrule opening. A terminal pin sealed in an insulator via hole has a first end that extends outwardly beyond an insulator device side. A filter capacitor adjacent to the insulator device side has a dielectric supporting interleaved active and ground electrode plates. A passageway extending through the dielectric has an internal metallization. An external metallization is on a terminated portion as opposed to an unterminated portion of the dielectric outer surface. The capacitor ground electrode plates extend to the external metallization at the terminated portion, but they do not extend to the unterminated outer surface portion. The outwardly extending terminal pin end is connected to the internal metallization in the dielectric passageway which in turn is connected to the active electrode plates. A conductive material connects the capacitor external metallization at the terminated dielectric outer surface portion to a system ground.

## Description

### CROSS-REFERENCE TO RELATED APPLICATION

This application claims priority to U.S. provisional patent application Serial No. 63/595,511, filed on November 2, 2023, and to U.S. patent application Serial No. 18/921,348, filed on October 21, 2024.

### FIELD OF THE INVENTION

The present invention generally relates to active implantable medical devices (AIMDs) including hermetic EMI filtered terminal pin subassemblies. In general, the present invention relates to an EMI filter capacitor, preferably having a rectangular shape, that is grounded on only one of its long sides. The opposed long side of the filter capacitor is left ungrounded. A single-sided system ground helps to reduce the complexity and cost associated with building an EMI filter capacitor, among other benefits. For asymmetrical hermetically-sealed terminal pins relative to the longitudinal axis of a ferrule, a single-sided ground metallization on the EMI filter capacitor also improves reliability and safety factor.

AIMDs, and in particular cardiac implantable electronic devices (CIEDs), have evolved over time to include higher and higher implantable lead counts. In the early days of pacemakers, only the right ventricle was paced. However, design improvements led to dual chamber pacing where the right ventricle and the right atrium were paced. This required two bipolar leads connected to four hermetically-sealed feedthrough terminal pins. At that time with only four terminal pins, it was practical to manufacture round quad-polar filtered hermetic seals.

However, modern CIEDs provide therapeutic pacing and biologic signal sensing to chambers in both the right and left sides of the heart. These are known as cardiac resynchronization devices. CRT-P devices are cardiac resynchronization therapy-pacemaker devices. CRT-D devices are cardiac resynchronization therapy-defibrillator devices. These resynchronization devices have hermetic seals with generally more than eight feedthrough terminal pins, for example, 11, 13, 16, and more terminal pins. Neurostimulators, such as spinal cord stimulators, generally have greater than 25 to 35 hermetically-sealed terminal pins.

But for patient comfort in the pectoral pocket, for example, it is very important that an AIMD be as thin as possible. As the number of hermetically-sealed feedthrough terminal pins that are connectable to associated implantable leads has increased over time, a round hermetic seal geometry is no longer desirable or practical. That is because round high terminal pin count filtered hermetic seals are much too large in diameter for acceptable patient comfort. For that reason, most present-day EMI filter capacitors for hermetic feedthroughs are rectangular with the terminal pins often aligned inline or in dual inline configurations to achieve both the required high terminal pin count and the desired device thinness.

Accordingly, the present invention relates to a novel design for an EMI filter capacitor for a hermetic feedthrough, preferably with the capacitor having a rectangular shape, and where the capacitor is only terminated on one of its long sides. The opposed long side of the rectangularly-shaped filter capacitor is left unterminated. The present one- or single-side system ground for an EMI filter capacitor offers a number of important manufacturing and cost reduction advantages as compared to the prior art double side terminated or even four-side terminated EMI filter capacitors.

### BACKGROUND OF THE INVENTION

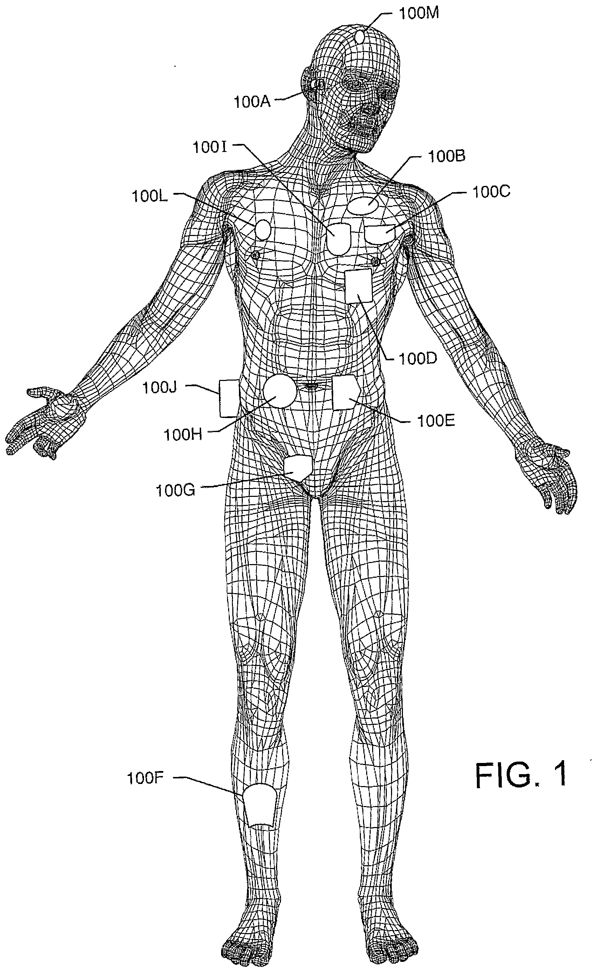

Turning now to the drawings, FIG. 1 is a wire form diagram of a generic human body illustrating various types of active implantable and external medical devices that include an EMI filter capacitor having a one- or single-side system ground according to the present invention and that can either be implanted in a patient's body or attached externally to the body.

Numerical designation 100A represents a family of hearing devices which can include the group of cochlear implants, piezoelectric sound bridge transducers, and the like.

Numerical designation 100B represents a variety of neurostimulators, brain stimulators, and brain sensors. Neurostimulators are used to stimulate the Vagus nerve, for example, to treat epilepsy, obesity, and depression. Brain stimulators are pacemaker-like devices and include electrodes implanted deep into the brain for sensing the onset of a seizure and also for providing electrical stimulation to brain tissue to prevent a seizure from actually occurring. Sensors include optical sensors, motion sensors, acoustic sensors, pressure sensors, analyte sensors, and electromagnetic sensors, among others.

Numerical designation 100C shows a cardiac pacemaker which is well-known in the art.

Numerical designation 100D includes the family of left ventricular assist devices (LVADs) and artificial heart devices.

Numerical designation 100E includes a family of drug pumps, which can be used for dispensing insulin, chemotherapy drugs, pain medications, and the like.

Numerical designation 100F includes a variety of bone growth stimulators for rapid healing of fractures.

Numerical designation 100G includes urinary incontinence devices.

Numerical designation 100H includes the family of pain relief spinal cord stimulators and anti-tremor stimulators. Numerical designation 100H also includes an entire family of other types of neurostimulators used to block pain.

Numerical designation 100I includes both implantable cardioverter defibrillator (ICD) devices and congestive heart failure devices (CHF). These are known in the art as cardio resynchronization therapy devices, otherwise known as CRT devices.

Numerical designation 100J illustrates an externally worn pack. The pack can be an external insulin pump, an external drug pump, an external neurostimulator or even a ventricular assist device.

Numerical designation 100K illustrates one of various types of EKG/ECG external skin electrodes which can be placed at various external locations on the body.

Numerical designation 100L represents external EEG electrodes that are placed on the head.

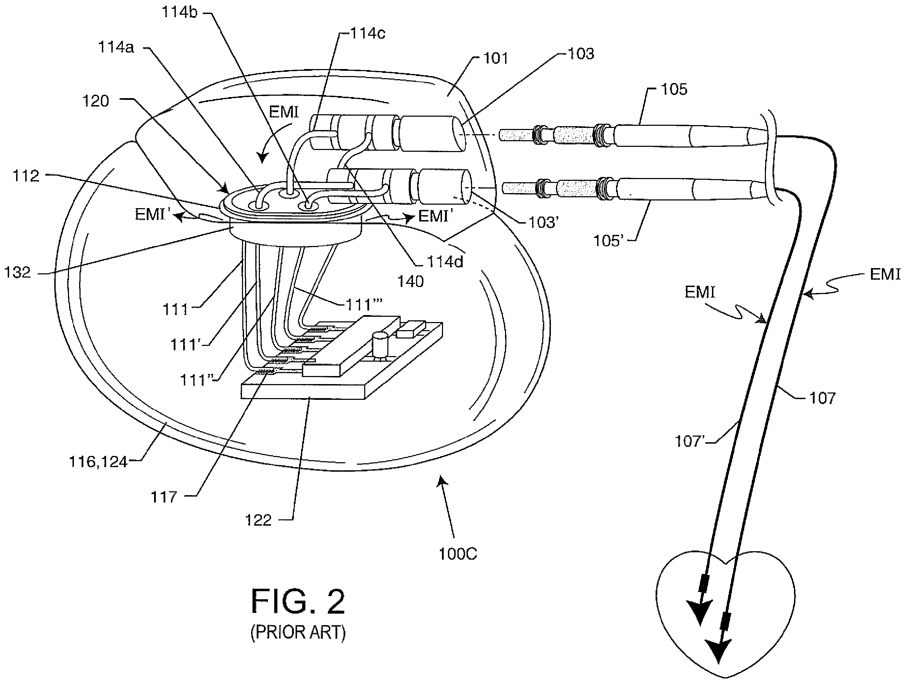

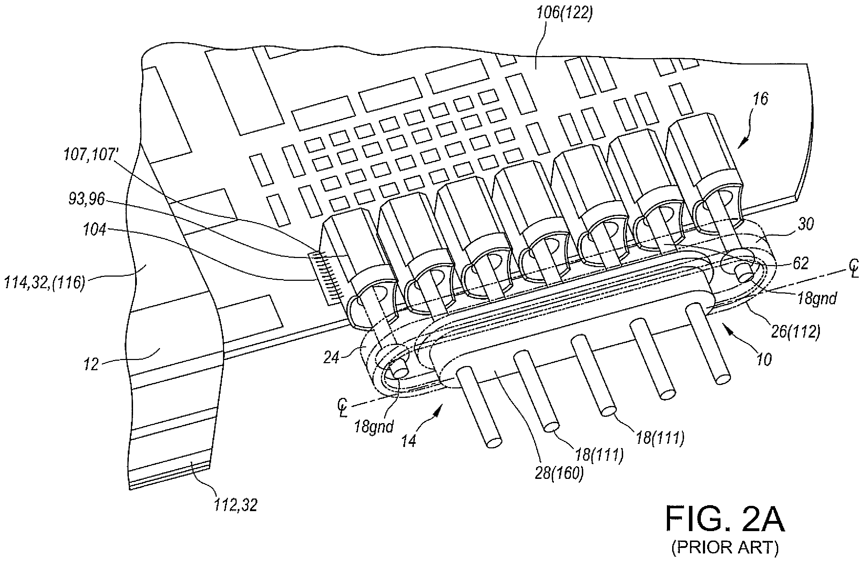

FIG. 2 is a side cutaway view of a prior art cardiac pacemaker 100C system. The pacemaker electronics reside inside a hermetically sealed AIMD housing 116 (typically made from titanium), which provides an electrically conductive electromagnetic shield. The header block 101 is generally made from a thermosetting insulating plastic or compound, such as Tecothane^{®}, and houses one or more connector assemblies, generally in accordance with ISO Standards IS-1, IS-2, IS4 or DF4. The header block port connector cavities (female connectors) are labelled 103, 103'. Implantable terminal pins 107, 107' have proximal plugs 105, 105' (male connectors), which are designed to be inserted into and mate with the female header block connector cavities 103, 103'. In devices that do not have header blocks, the implantable terminal pins are built directly into the pulse generator itself.

Further regarding FIG. 2, the system ground 124 comprises the conductive AIMD housing 116, which, as noted above, provides the overall electromagnetic shield, and also functions as an energy dissipating surface. The AIMD also has a hermetically sealed feedthrough 120 to which a quad-polar EMI filter capacitor 132 is mounted. Since the conductive ferrule 112 of the feedthrough 120 is electrically connected to the AIMD housing 116, the ferrule 112 is also part of the system ground 124. Accordingly, the system ground 124 illustrated in FIG. 2 includes the ferrule 112 and the device housing 116.

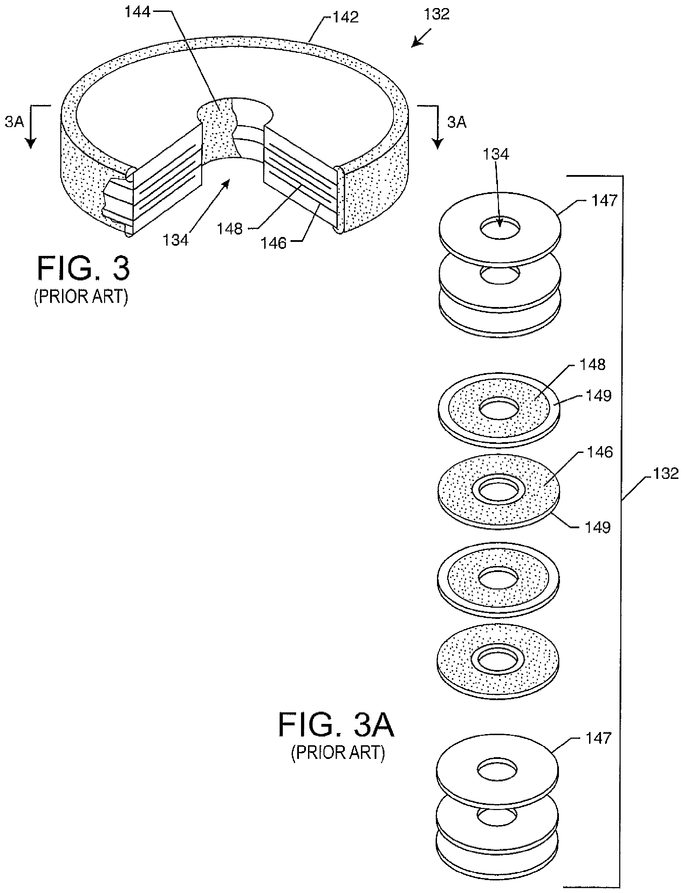

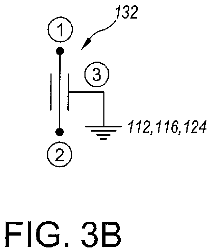

Still referring to FIG. 2, the electrical connection of the EMI filter capacitor 132 to the system ground 124 is better appreciated. When the external ground metallization 142 of the EMI filter capacitor 132 is electrically connected to the ferrule 112 of the hermetically sealed feedthrough 120, and the ferrule 112 in turn is hermetically sealed (typically by welding, such as laser welding) to the conductive housing 116 of an AIMD, for example, a cardiac pacemaker 100C, then the external ground metallization 142 (FIG. 3) is electrically connected to the system ground 124. It is also within the scope of the present invention that the ferrule 112 can also be a continuous part of the device housing 116. This means that the ground metallization 142, the ferrule 112 and the AIMD housing 116 are all at the same potential; they are all at ground potential and are all part of the overall AIMD equipotential surface (in other words, system ground 124). Since the hermetically sealed enclosure of the AIMD blocks EMI from entering inside the device housing 116, it is commonly known as an EMI shield or a Faraday cage.

Additionally, the EMI filter capacitor 132 is designed to selectively redirect undesirable high-frequency energy before it enters the AIMD housing 116 to this equipotential surface for diversion and/or energy dissipation. Ideally, the role of the EMI filter capacitor 132 is to allow low frequency biologic signals, such as therapeutic pacing pulses, to freely pass, without attenuation, while at the same time diverting dangerous high-frequency EMI energy to the AIMD housing 116 comprising the equipotential surface. When the undesirable EMI energy is diverted to the AIMD housing 116, that energy is dissipated harmlessly as a few milliwatts of heat energy. In this manner, dangerous EMI energy is prevented from entering the AIMD housing 116 where it could reach sensitive AIMD circuitry and seriously disrupt the proper operation of any one of the above-described medical devices 100A to 100L. Such EMI disruption could inadvertently suspend therapy, which, depending on the medical device, could be immediately life threatening.

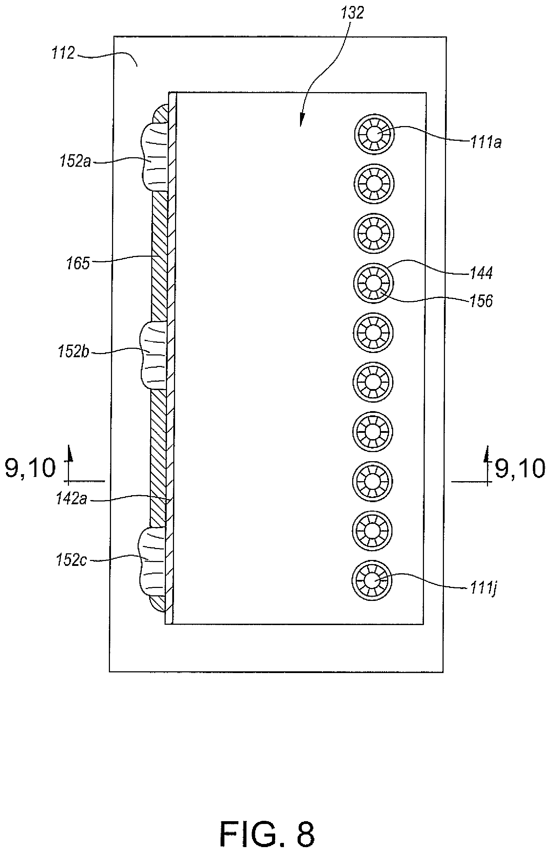

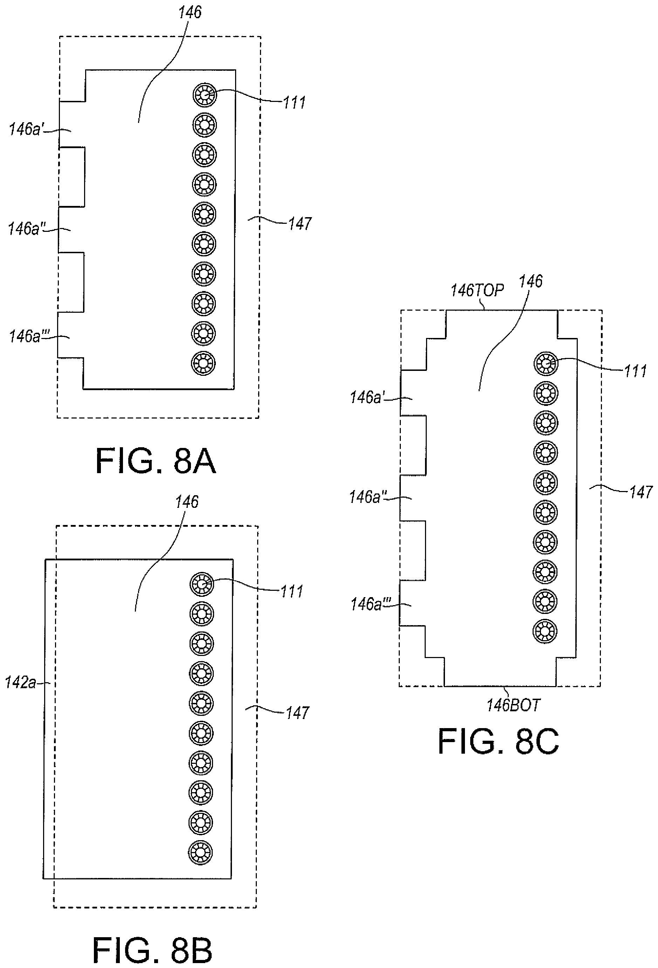

FIG. 2A is a cutaway view of a prior art CIED showing an AIMD circuit board 106 (122) with surface mounted terminal pin connectors 16. Terminal pin connectors 16 are taught by U.S. Patent No. 11,211,741, which is assigned to the assignee of the present invention and incorporated herein by reference. FIG. 2A herein is adapted from FIG. 8 of the `741 patent with the element numbers shown in the drawing being taken from that patent, but with the number in parentheses being consistent with the element numbers in the other drawing figures of the present patent application. In that respect, FIG. 2A illustrates that the hermetically-sealed terminal pins 18 (111) are off-center with respect to a center line of the ferrule 26 (112), which is also known as the ferrule longitudinal axis. The reason for this is that the terminal pin connectors 16 are substantially elevated above the AIMD circuit board 106 (122) so that the off-center hermetically-sealed terminal pins 18 (111) align with the terminal pin connectors 16 and can be easily plugged into the connectors. As will be shown, the present invention is particularly useful for hermetically-sealed terminal pins 18 (111) that are off-center with respect to the ferrule longitudinal axis or center line.

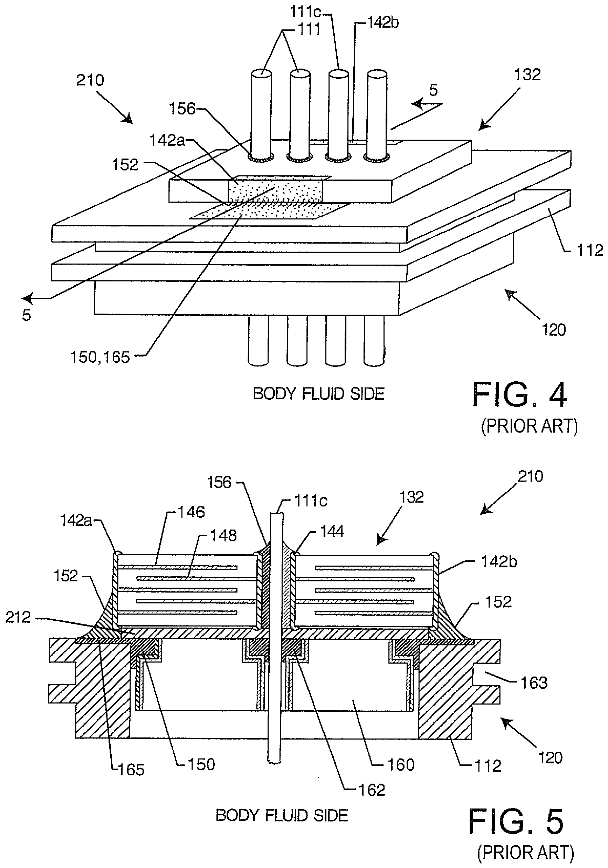

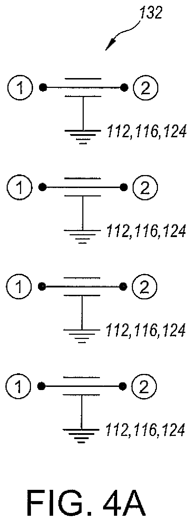

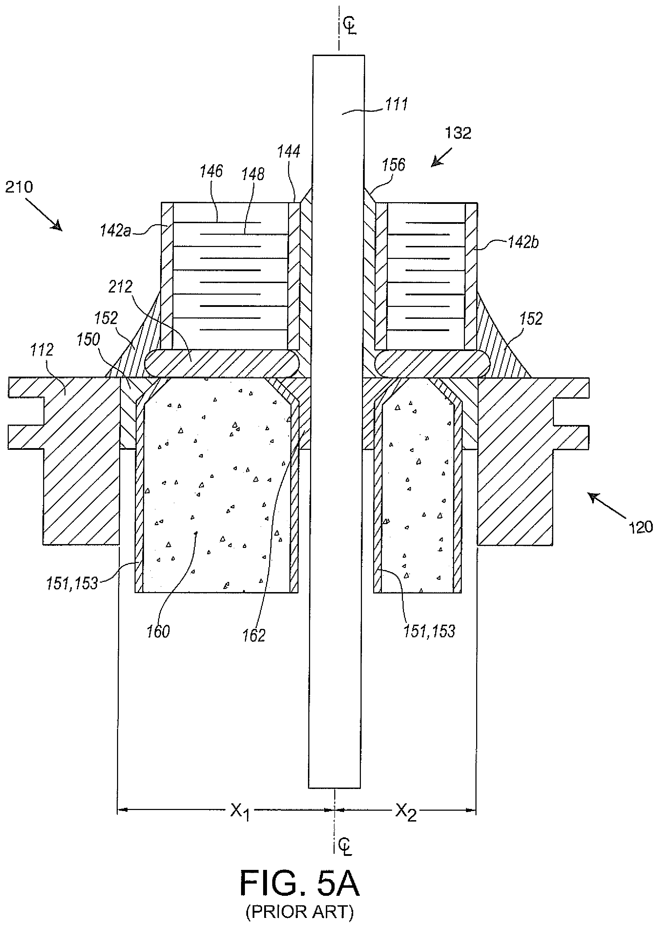

FIG. 3 is an isometric cutaway view of a prior art round unipolar (one capacitor passageway 134) feedthrough EMI filter capacitor 132. As previously described, round AIMD EMI filtered hermetic seals are no longer desirable due the current high terminal pin counts and the desired thinness of implantable medical devices for patient comfort. The unipolar EMI filter capacitor has an external ground metallization 142 and a metallization 144 at the active passageway 134. These metallizations can be applied by electroplating, physical vapor deposition or glass frit metallization bonding. In one embodiment, the capacitor metallizations may comprise silver, copper, platinum, palladium, platinum silver, palladium silver, and combinations thereof. After the external ground metallization 142 and the passageway active metallization 144 are applied, the EMI filter capacitor 132 is electrically connected to a hermetically sealed feedthrough 120 using an electrical connection material 152 (FIGs. 4 and 5) such as a solder, a thermosetting electrically conductive adhesive, an electrically conductive silicone, an electrically conductive polyimide, an electrically conductive epoxy, and the like. As illustrated, the unipolar EMI filter capacitor 132 comprises active electrode plates 148 and ground electrode plates 146. The ground electrode plates 146 are electrically connected in parallel to each other through the external ground metallization 142 and the active electrode plates 148 are electrically connected in parallel to each other through the passageway metallization 144.

FIG. 3A is an isometric, exploded view of the prior art feedthrough EMI filter capacitor 132 shown in FIG. 3. Active electrode plates 148 are disposed on ceramic substrates 149 to form an active electrode layer, and the ground electrode plates 146 are disposed on ceramic substrates 149 to form a ground electrode layer. The active and ground electrode plates 148, 146 are interleaved and stacked one upon the other, with one or more ceramic cover sheets 147 positioned at the top and bottom of the stack. The stack is then pressed and laminated. It is appreciated that blank ceramic cover sheets 147 can be disposed between the active and ground electrode plates 148, 146 to increase the dielectric thickness and the voltage rating of the device. The electrode plates 146 and 148 are typically applied by silk-screening or equivalent waterfall processes.

FIG. 3B is the schematic diagram for the unipolar filter shown in FIG. 3. System ground is indicated with numerical designation 124 and can comprise the ferrule 112, the housing 116, or both.

FIG. 4 illustrates a prior art inline quad-polar EMI filter capacitor 132 mounted on a hermetically sealed feedthrough to thereby form a filtered feedthrough 210. Opposed electrical connection materials 152 electrically connect the ground metallizations 142a and 142b to the ferrule 112, which is generally of titanium. The ground electrical connection materials 152 are desirably very low impedance and very low resistance. The two ground electrical connection materials 152 are connected to respective gold bond pads 150, 165, which form oxide resistant electrical connections to the ferrule 112. Oxide resistant EMI filter connections are taught by U.S. Patent No. 6,765,779, which is assigned to the assignee of the present invention and incorporated herein fully by reference. U.S. Patent Nos. 9,427,596, 9,931,514, 10,350,421, 11,241,581, 11,344,734, and 11,633,612 also teach oxide resistant low impedance electrical connections to a titanium ferrule. These patents are also assigned to the assignee of the present invention and fully incorporated herein by reference.

Connection material 156 electrically connects the capacitor passageway active metallizations 144 (FIG. 3) directly to the terminal pins 111.

FIG. 4A is the electrical schematic of the prior art quad-polar EMI filter capacitor shown in FIG. 4.

FIG. 5 is a cross-sectional view generally taken along line 5-5 of FIG. 4. This drawing shows gold braze 162 hermetically sealing terminal pin 111, which is representative of the terminal pins 111, 111', 111'' and 111‴ shown in FIG. 2 and the terminal pins 111 and 111c shown in FIG. 4, to the feedthrough insulator 160. Gold braze 150 hermetically seals the feedthrough insulator 160 to the ferrule 112.

FIG. 5A is similar to FIG. 5 except that the hermetically-sealed terminal pin 111 is not symmetrical with respect to the longitudinal axis of the ferrule 112. The reason for this asymmetry was previously explained with respect to FIG. 2A regarding alignment with the AIMD circuit board 106 (122) terminal pin connectors 16.

### SUMMARY OF THE INVENTION

Aspects and embodiments of the present invention are set out in the appended claims.

The present invention relates to a hermetically sealed filtered feedthrough for an active implantable medical device (AIMD). In one embodiment, the filtered feedthrough comprises an electrically conductive ferrule having a ferrule opening extending to spaced-apart ferrule device and body fluid sides. Preferably, the ferrule has a rectangular shape so that in plan view looking at either of the ferrule device side or body fluid sides, the ferrule comprises opposed ferrule first and second longitudinal side walls that extend to and meet with opposed ferrule third and fourth end walls with the longitudinal side walls being longer than the end walls. The first and second longitudinal side walls are preferably aligned parallel to and on opposite sides of a ferrule center line that bisects the opposed third and fourth end walls.

An electrically non-conductive insulator has an insulator outer surface that extends to spaced-apart insulator device and body fluid sides. The insulator disposed in the ferrule opening is hermetically sealed to the ferrule by a first gold braze so that when the ferrule hermetically sealed to the insulator is attached to an opening in a housing of an AIMD, the ferrule and insulator body fluid sides, and the corresponding ferrule and insulator device sides reside outside and inside the AIMD, respectively.

Further, at least two insulator via holes extending to the insulator device and body fluid sides may reside between the insulator second longitudinal side wall and the ferrule center line, and an insulator metallization is disposed on the insulator outer surface and in the insulator via holes. A respective one of at least two terminal pins reside in one of the insulator via holes where a second gold braze hermetically seals the terminal pin to the insulator. The terminal pins extend to terminal pin first and second ends with at least the terminal pin first ends extending outwardly beyond the insulator device side. That way, the at least two terminal pins reside between the insulator second longitudinal side wall and the ferrule center line.

A filter capacitor is disposed at or adjacent to the insulator device side. The capacitor comprises a dielectric outer surface extending to a dielectric first major face spaced from a dielectric second major face. At least one active electrode plate and at least one ground electrode plate are supported in the capacitor dielectric in an interleaved, partially overlapping capacitive relationship. Then, preferably at least two dielectric passageways extend to the dielectric first and second major faces and a capacitor internal metallization is disposed in the dielectric passageways. The at least one active electrode plate is connected to the capacitor internal metallization in the dielectric passageways, and the outwardly extending terminal pin first ends reside in a respective one of the dielectric passageways where the terminal pin is conductively connected to the capacitor internal metallization connected to the at least one active electrode plate by a first conductive material. Moreover, the at least one ground electrode plate is in a non-conductive relation with the capacitor internal metallization in the dielectric passageway.

A capacitor external metallization is disposed on a terminated outer surface portion, but not on an unterminated outer surface portion of the dielectric outer surface. Importantly, the at least one ground electrode plate is conductively connected to the capacitor external metallization at the terminated outer surface portion with the at least one active electrode plate being in a non-conductive relation with the capacitor external metallization. A second conductive material connects the capacitor external metallization at the terminated dielectric outer surface portion to the ferrule or to the first braze sealing the insulator to the ferrule to provide a system ground for the filtered feedthrough.

In another embodiment of the present filtered feedthrough, the filter capacitor disposed at or adjacent to the insulator device side comprises a capacitor dielectric having a dielectric outer surface extending to a dielectric first major face spaced from a dielectric second major face. In plan view looking at either of the dielectric first major face or the second major face, the capacitor dielectric comprises opposed dielectric first and second long sides extending to and meeting with opposed dielectric third and fourth short ends. The dielectric long sides are longer than the dielectric short ends.

At least one active electrode plate and at least one ground electrode plate are supported in the capacitor dielectric in an interleaved, partially overlapping relationship. A dielectric passageway extends to the dielectric first and second major faces and a capacitor internal metallization is disposed in the dielectric passageway. The at least one active electrode plate is connected to the capacitor internal metallization in the dielectric passageway with the outwardly extending terminal pin first end residing in the dielectric passageway where the terminal pin is conductively connected to the capacitor internal metallization connected to the at least one active electrode plate by a first conductive material. However, the at least one ground electrode plate is in a non-conductive relation with the capacitor internal metallization in the dielectric passageway.

A capacitor external metallization is disposed on a terminated outer surface portion, but not on an unterminated outer surface portion of the dielectric outer surface. Then, the at least one ground electrode plate extends to the dielectric first long side comprising the terminated dielectric outer surface portion, but the ground electrode plate does not extend to the second long side comprising the unterminated outer surface portion of the capacitor dielectric so that the at least one ground electrode plate is conductively connected to the capacitor external metallization at the dielectric first long side as the terminated dielectric outer surface portion. A second conductive material connects the capacitor external metallization at the terminated dielectric outer surface portion to the ferrule or to the first braze sealing the insulator to the ferrule to provide a system ground for the filtered feedthrough. The at least one active electrode plate is in a non-conductive relation with the capacitor external metallization.

In a further embodiment of the present filtered feedthrough, the filter capacitor disposed at or adjacent to the insulator device side comprises a capacitor dielectric having a dielectric outer surface extending to a dielectric first major face spaced from a dielectric second major face. In plan view looking at either of the dielectric first major face or the second major face, the capacitor dielectric comprises opposed dielectric first and second long sides extending to and meeting with opposed dielectric third and fourth short ends. The dielectric long sides are longer than the dielectric short ends.

At least one active electrode plate and at least one ground electrode plate are supported in the capacitor dielectric in an interleaved, partially overlapping relationship. A dielectric passageway extends to the dielectric first and second major faces and a capacitor internal metallization is disposed in the dielectric passageway. The at least one active electrode plate is connected to the capacitor internal metallization in the dielectric passageway with the outwardly extending terminal pin first end residing in the dielectric passageway where the terminal pin is conductively connected to the capacitor internal metallization connected to the at least one active electrode plate by a first conductive material. However, the at least one ground electrode plate is in a non-conductive relation with the capacitor internal metallization in the dielectric passageway.

A capacitor external metallization is disposed on the dielectric first long side as a terminated outer surface portion, but not on the dielectric second long side as an unterminated outer surface portion. Then, the at least one ground electrode plate extends to the dielectric first long side comprising the terminated dielectric outer surface portion, but the ground electrode plate does not extend to the second long side comprising the unterminated outer surface portion of the capacitor dielectric. Moreover, the dielectric first long side is spaced above the first gold braze hermetically sealing the insulator to the ferrule, but the dielectric second long side extends laterally outwardly beyond the first gold braze. Then, a second conductive material connects the capacitor external metallization at the dielectric first long side to the ferrule or to the first braze sealing the insulator to the ferrule to provide a system ground for the filtered feedthrough. The at least one active electrode plate is in a non-conductive relation with the capacitor external metallization.

In a still further embodiment of the present filtered feedthrough, the capacitor external metallization is disposed on at least a portion of the dielectric outer surface to provide a terminated dielectric outer surface portion. In this embodiment, the ground electrode plate extends to a first segment of the terminated dielectric outer surface portion, but the ground electrode plate does not extend to a second segment of the terminated dielectric outer surface portion. Then a second conductive material connects the capacitor external metallization on the dielectric outer surface portion to the ferrule or to the first braze sealing the insulator to the ferrule to provide a system ground for the filtered feedthrough. The at least one active electrode plate is in a non-conductive relation with the capacitor external metallization.

Further regarding this embodiment of the present invention, in plan view looking at either of the dielectric first major face or the second major face, the capacitor dielectric has a rectangular shape comprising opposed dielectric first and second long sides extending to and meeting with opposed dielectric third and fourth short ends. The capacitor the external metallization is disposed on both of the dielectric long sides comprising the terminated dielectric outer surface portion, and the ground electrode plate extends to the first long side of the capacitor dielectric as the first segment of the terminated dielectric outer surface portion, but the ground electrode plate does not extend to the second long side of the capacitor dielectric as the second segment of the terminated dielectric outer surface portion. Further, the dielectric third and fourth short ends are also provided with the external metallization and the ground electrode plate either does or does not extend to the third and fourth short ends of the capacitor dielectric outer surface.

These and other objects of the present invention will become increasingly more apparent to those skilled in the art by reference to the following description and to the appended drawings.

### BRIEF DESCRIPTION OF THE DRAWINGS

FIG. 1 is a wire formed diagram of a generic human body showing a number of medical devices 100A to 100L according to the present invention that can either be implanted in a patient's body tissue or attached externally to the body.
FIG. 2 is a side cutaway view of a prior art cardiac pacemaker 100C system.
FIG. 2A is a cutaway view of a prior art cardiac implantable electronic devices (CIED) showing an AIMD circuit board 106 (122) with surface mounted terminal pin connectors 16.
FIG. 3 is an isometric cutaway view of a prior art round unipolar (one capacitor passageway 134) feedthrough EMI filter capacitor 132.
FIG. 3A is an isometric, exploded view of the feedthrough EMI filter capacitor 132 shown in FIG. 3.
FIG. 3B is the schematic diagram for the unipolar filter shown in FIG. 3.
FIG. 4 is a perspective view of a prior art inline quad-polar EMI filter capacitor 132 mounted on a hermetically sealed feedthrough 120 to thereby form a filtered feedthrough 210.
FIG. 4A is the electrical schematic of the quad-polar EMI filter capacitor shown in FIG. 4.
FIG. 5 is a cross-sectional view taken along line 5-5 of FIG. 4.
FIG. 5A is a cross-sectional view an EMI filter capacitor 132 mounted on a hermetically sealed feedthrough 120 to thereby form a filtered feedthrough 210 similar to the filtered feedthrough shown in FIGs. 4 and 4A except that the hermetically-sealed terminal pin 111 is not symmetrical with respect to the longitudinal axis of the ferrule 112.
FIG. 6 is a perspective view of an inline quad-polar EMI filter capacitor 132 mounted on a hermetically sealed feedthrough 120 to thereby form a filtered feedthrough 210 similar that shown in FIG. 4, except that the back-side or right-side ground metallization 142b for the EMI filter capacitor 132 has been eliminated according to the present invention.
FIG. 6A is the electrical schematic of the quad-polar EMI filter capacitor shown in FIG. 6.
FIG. 7 is a cross-sectional view taken along line 7-7 of FIG. 6.
FIG. 8 is a cross-sectional view taken along line 8-8 of FIG 6.
FIG. 8A is a plan view of one of the ground electrode plates 146 of the ten-pole EMI filter capacitor shown in FIG. 8.
FIG. 8B is a plan view that is similar to FIG. 8A except that the ground metallization 142a connected to the ground electrode plates 146 extends along the entire length of the left side of the capacitor dielectric 147, which defines the left edge or left side of the EMI filter capacitor 132.
FIG. 8C is a plan view similar to FIG. 8A except that the ground electrode plates 146 also extend to the top 146TOP and to the bottom 146BOT of the EMI filter capacitor 132.
FIG. 9 is a cross-sectional view taken along line 9-9 of FIG. 8.
FIG. 9A is a cross-sectional view similar to the view shown in FIG. 9 except that the ground electrode plates 146, but not the active electrode plates 148, extend to the unterminated edge of the capacitor dielectric 147.
FIG. 9B is a cross-sectional view similar to the view shown in FIG. 9 except that the active electrode plates 148, but not the ground electrode plates 146, extend to the unterminated edge of the capacitor dielectric 147.
FIG. 9C is a cross-sectional view similar to the view shown in FIG. 9 except that both the active and ground electrode plates 146, 148 extend to the unterminated edge of the capacitor dielectric 147.
FIG. 9D is a cross-sectional view similar to the view shown in FIG. 9C with both the active and ground electrode plates 146, 148 extending to the unterminated edge of the capacitor dielectric 147, and with an insulative material 213 contacting the exposed edges of the opposite polarity electrode plates.
FIG. 9E is a cross-sectional view similar to the view shown in FIG. 9C with the entire outer surface of the capacitor dielectric 147 supporting an external ground metallization 142a, but with the ground electrode plates 146 only extending to the external metallization on the left edge but not to the external metallization on the right edge of the capacitor dielectric 147.
FIG. 10 is a cross-sectional view taken along line 10-10 of FIG. 8 except that the active and ground electrode plates are shown as a closely-spaced pair of active electrode plates and a closely-spaced pair of ground electrode plates.
FIG. 11 illustrates an EMI filter circuit board 155 with a single-sided grounded electrical connection material 152/gold braze 250 connection to the ferrule 112.

### DETAILED DESCRIPTION OF THE PREFERRED EMBODIMENTS

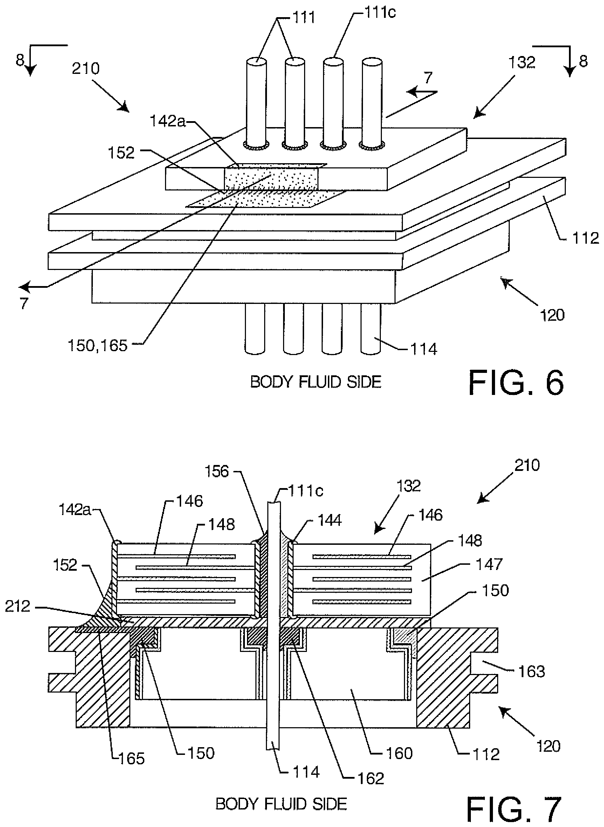

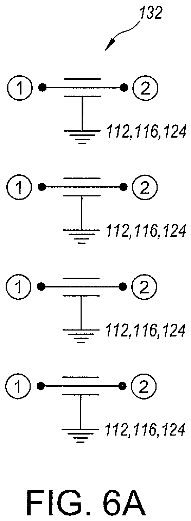

Turning now to FIG. 6, this drawing is similar to FIG. 4, except that the back-side ground metallization 142b for the EMI filter capacitor 132 has been eliminated. This means that the only electrical connection to system ground is at the front-side ground metallization 142a, which forms a single-sided system ground. In this case, the terminal pins 111, 111c are symmetrical with the longitudinal axis of the ferrule.

FIG. 7 is a cross-sectional view taken along line 7-7 of FIG. 6. In comparison to FIG. 5, the capacitor ground metallization 142a is connected through an electrical connection material 152 to the system ground 165, 150. Numerical designation 165 refers to a gold pad that is contained in a ferrule pocket and numerical designation 150 illustrates the gold braze that hermetically seals the insulator 160 to the conductive ferrule 112. In a preferred embodiment, the electrical connection material 152 is a thermal-setting conductive polymer, such as a conductive epoxy, a polyimide, a solder, and the like.

Referring still to FIG. 7, the right-side ground metallization 142b (or the back-side ground termination in FIG. 6) and the corresponding electrical connection material 152 on the right side of the EMI filter capacitor 132 shown in FIGs. 5 and 5A has been eliminated. Moreover, there is still a hermetic seal 150 between the insulator 160 and the ferrule 112 in this cross-sectional view on the right-side of the EMI filter capacitor 132, but the gold pad 165 is no longer needed on the right side because there is no electrical connection material 152 on that side of the EMI filter capacitor 132. Elimination of the right-side ground metallization 142b and its associated electrical connection material 152 reduces manufacturing steps and costs. In FIG. 7, the ground electrode plates 146 extend on the left-hand side all the way to the capacitor ground metallization 142a. However, on the right side, the ground electrode plates 146 stop short of the outside edge of the EMI filter capacitor 132 and the capacitor dielectric 147 insulates this entire area.

There are a number of advantages to the novel single-sided EMI filter capacitor ground, as illustrated in FIG. 7. They include reduced manufacturing costs by eliminating the right-side ground metallization 142b and its associated ground electrical connection material 152, and elimination of the rather large gold oxide-resistant contact pad 165. Also, eliminated is the associated labor attendant to the eliminated materials.

At first glance, it would seem that elimination of the right-side ground metallization 142b would undesirably degrade the filter performance of the EMI capacitor 132, which is known as insertion loss and is measured in dBs or decibels. Admittedly, there is both resistivity and inductance attributed to the ground and active electrode plates 146, 148. While there would be a slight increase in resistance, which shows up as a slightly higher equivalent series resistance (ESR) for the EMI filter capacitor 132, the increase in ESR is trivial, particularly, when there is a multiplicity of both active and ground electrode plates.

Inductance of the active and ground electrode plates is a little trickier to analyze. The inventor ran various plots and simulations using PSpice to analyze the effect of elimination of the right-side ground metallization 142b/electrical connection material 152 along one side of the EMI filter capacitor 132. The effective capacitance area, which is determined by the overlap of the ground and the active electrode plates 146, 148 along with their overlapping cross-sectional areas, does not change. Extension of the ground electrode plates 146 to the right side of the EMI filter capacitor 132 in FIG. 6 does not increase the ECA because this is a margin region in which there is no overlap of the ground and active electrode plates 146, 148. This means that the effective capacitance area and therefore the total capacitance is not changed.

What does change, however, is the inductance along the electrode plate. The inductive reactance X_{L} is equal to 2nfL (where L is the inductance and f is frequency). An easy way to analyze this is by examining the body fluid side of a terminal pin 111. The body fluid side is the outwardly extending end of a terminal pin that connects to the implanted leads 107, 107' (FIG. 2), for example, for a cardiac pacemaker or an implantable defibrillator. Electromagnetic (EMI) noise, such as the RF noise induced by a cellular telephone, can be picked up by these leads. EMI noise can travel up the leads 107, 107' to their respective terminal pin 111. However, before the electromagnetic noise can get into the interior of the AIMD, it is intercepted by the EMI filter capacitor 132. At low frequencies, the inductive reactance is trivial (the inductive reactance varies directly with frequency as shown in the above equation). So, at low frequencies, such as MRI RF pulsed frequencies (1.5 T is 64 MHz and 3T is 128 MHz) attenuation is unaffected. At very high frequencies, however, where the efficiency of the EMI filter capacitor on the right-hand side is somewhat reduced due to the inductance of the longer electrode plates 148, filter insertion loss is slightly degraded. That really does not matter because the high value of capacitance is not needed for effective attenuation at high frequencies. Therefore, the left-side of an EMI filter capacitor 132, as exemplified in FIG. 7, at frequencies above 200 MHz, for example, at 3 GHz, is still a very effective EMI filter capacitor whose performance has not been appreciably compromised.

Moreover, it has been shown that use of closely-spaced pairs of active electrode plates and closely-spaced pairs of ground electrode plates eliminates the increased inductance resulting in equivalent performance to a prior art dual sided ground. Closely-spaced pairs of active and ground electrode plates are thoroughly described by U.S. Patent No. 5,978,204 (Ex Parte Reexamination Certificate 4920^{th}), which is assigned to the assignee of the present invention and herein fully incorporated by reference.

In the present invention, with elimination of one of the two rectangular capacitor edge ground terminations, it is desirable to model each design to make sure it will have the required insertion loss and equivalent series resistance properties. Such modeling is well known to those skilled in the art of electromagnetic interference compatibility and EMI filter design, in particular.

FIG. 6A is the electrical schematic of the single-sided ground quad-polar EMI filter capacitor shown in FIG. 6. Note that this schematic is identical to the schematic of the prior art double sided ground quad-polar EMI filter capacitor of FIG. 4A (high frequency parasitic capacitance has been modeled and can be ignored in this case, particularly at MRI RF pulsed frequencies).

FIGURE 8 is a plan view looking at the device side of a 10-pole inline rectangular EMI filter capacitor 132. This plan view is suggested by the cross-sectional view taken along line 8-8 of FIG. 6, which looks down at the device side of the filtered feedthrough 210. As shown in FIG. 8, a ground metallization 142a is contacted to the left side of the ground plates 146 (FIG. 7). There is also an exposed gold braze 165 hermetically sealing the insulator to the ferrule 112. The expose gold braze 165 is required in order to make an oxide-resistant electrical connection. Spaced apart electrical connection materials 152a, 152b and 152c are shown connecting the EMI filter capacitor 132 to the EMI RF system ground. In a preferred embodiment, the electrical connection materials 152a, 152b and 152c are spaced-apart, but are continuous along the long side of the ground plates 146 and are contacted to the gold braze 162 and the ground metallization 142a. In accordance with the present invention, this drawing also illustrates that there is no termination material (prior art ground metallization 142b) on the right side of the EMI filter capacitor.

FIG. 8A illustrates one of the ground electrode plates 146 of the ten-pole EMI filter capacitor shown in FIG. 8. On the left side, the representative ground electrode plate 146 has plate extensions 146a', 146a'' and 146a‴ that extend to the left side of the capacitor dielectric 147. However, in accordance with the present invention, the ground electrode plate 146 does not extend to the right side of the capacitor dielectric 147.

FIG. 8B is similar to FIG. 8A except that the ground metallization 142a connected to the ground electrode plates, which are exemplified by ground plate 146, extends along the entire length of the left side of the capacitor dielectric 147, which defines the left edge or left side of the capacitor 132.

FIG. 8C is similar to FIG. 8A except that the ground electrode plates, which are exemplified by ground plate 146 also has top and bottom extensions that extend to the top 146TOP and to the bottom 146BOT edges of the capacitor dielectric 147. The addition of ground electrode plate extensions 146top and/or 146bot is still defined herein as a single-sided filter system ground connection as the effect on EMI filter performance (particularly for terminal pins 111 near the center terminal pin of the EMI filter capacitor 132) is trivial. Moreover, the single-sided filter system ground connection is applicable to the construction shown in FIG. 8C because the ground electrode plate 146 does not extend to the right side of the capacitor dielectric 147. The addition of ground electrode plate extensions 146top and/or 146bot is thereby not considered a "work around" to the present single-sided system EMI filter ground connection.

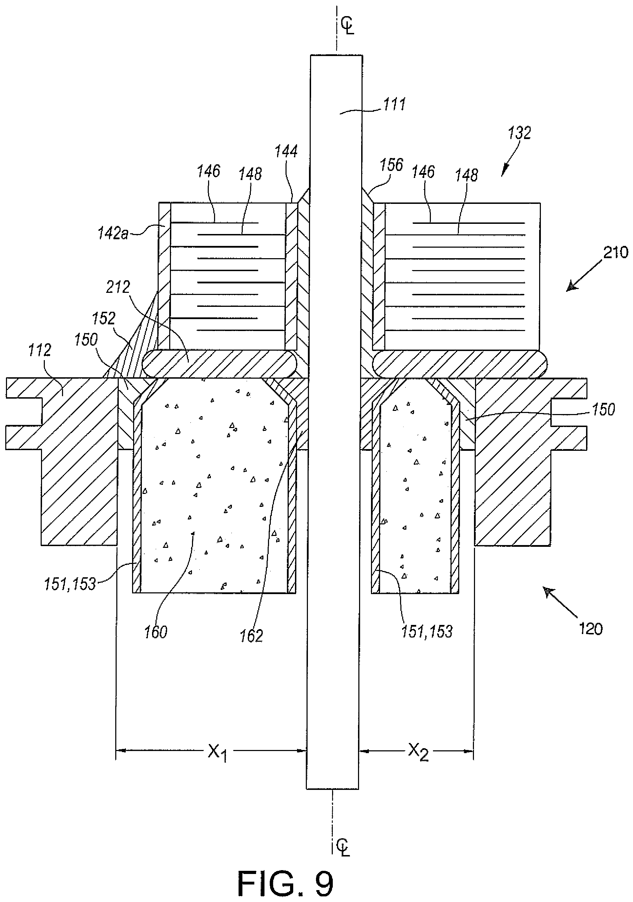

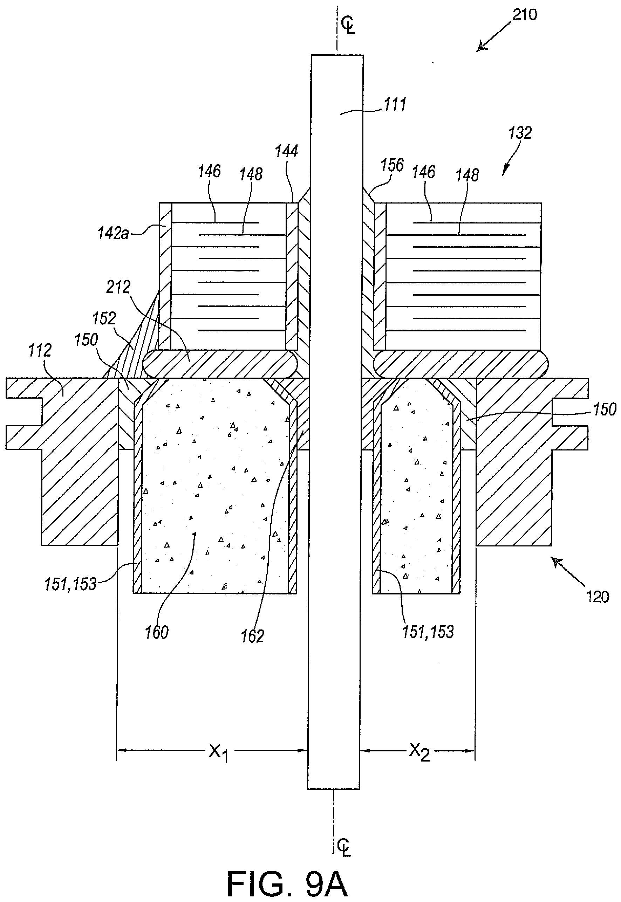

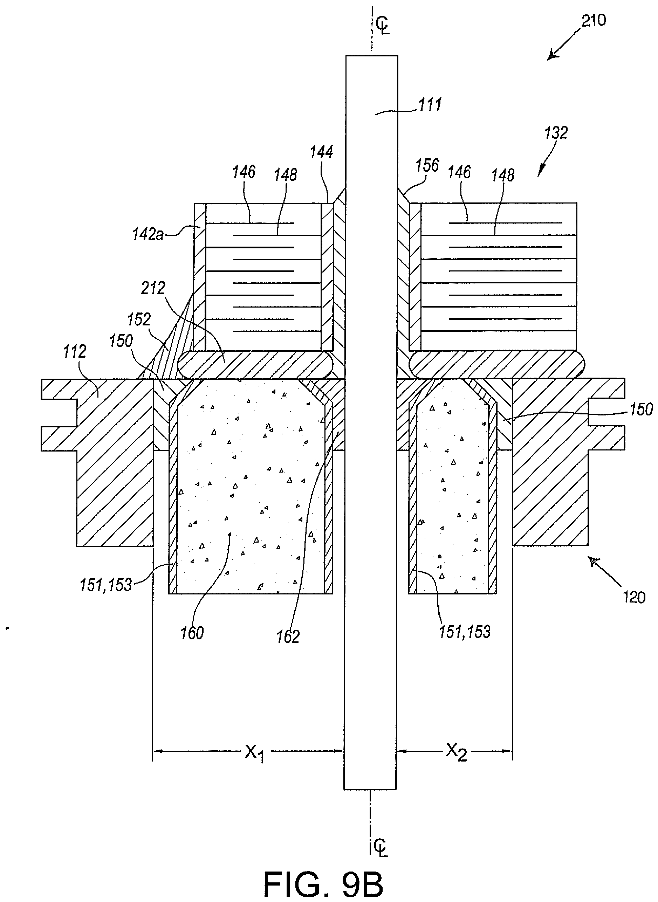

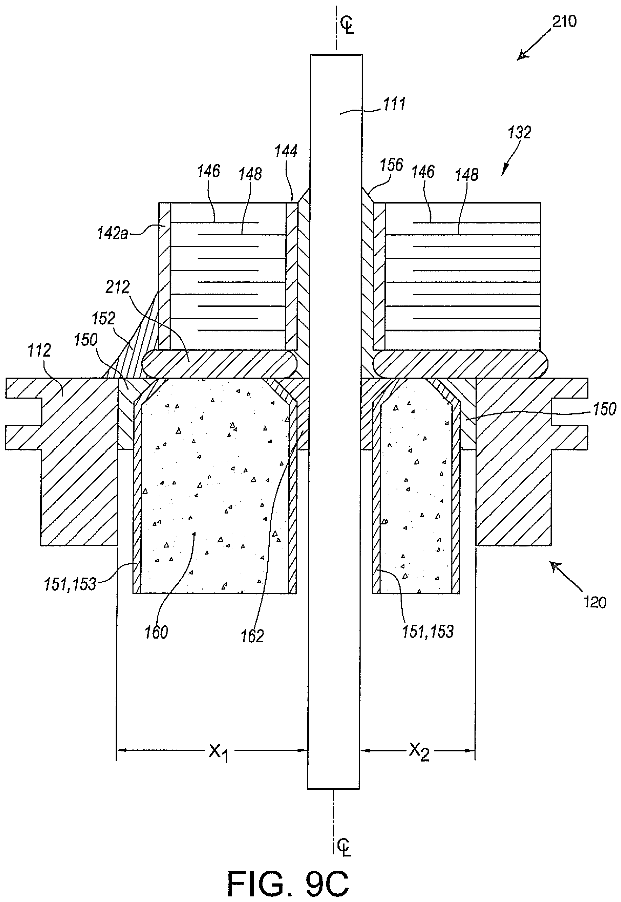

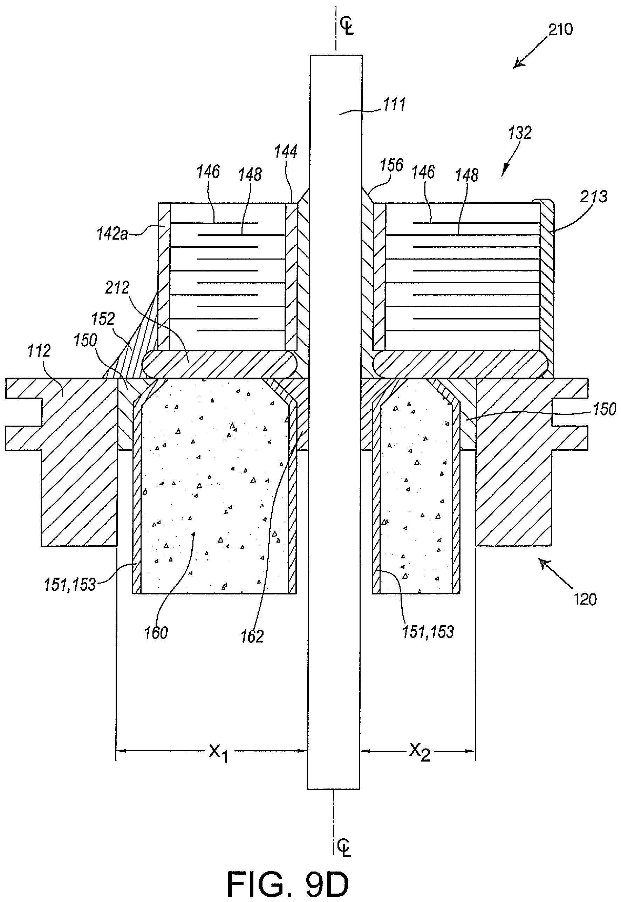

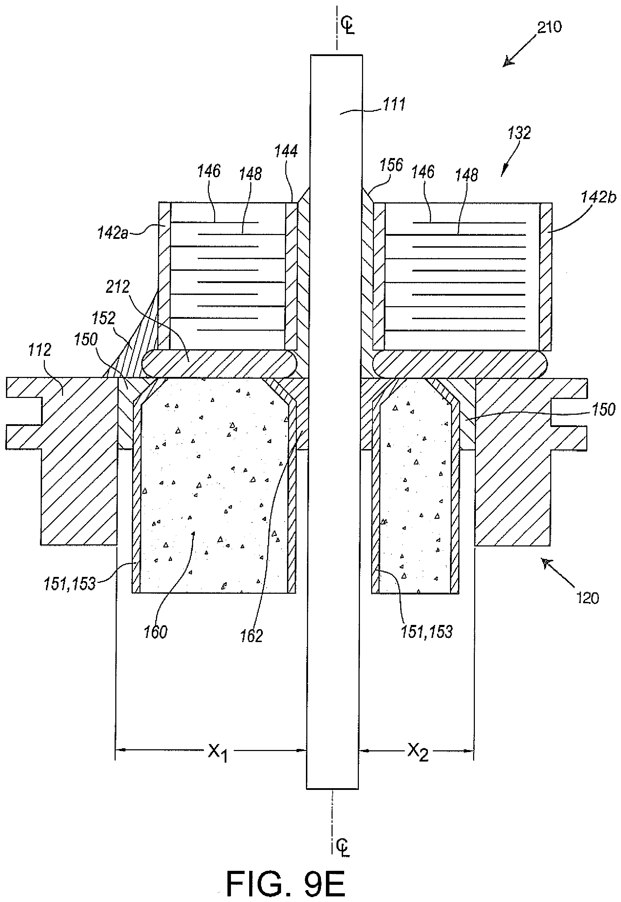

FIGURE 9 is a cross-sectional view taken along line 9-9 of FIG. 8 of another embodiment of a filtered feedthrough according to the present invention. In this embodiment X₁ is greater than X₂, which means that the terminal pins 111 (111a to 111j) are asymmetrical with respect to the longitudinal axis of the ferrule 112. Referring to this cross-sectional view, one can clearly see the electrical connection material 152 connecting the ground metallization 142a to the gold braze 150 of the hermetic terminal on the left side of the EMI filter capacitor 132. As previously mentioned, contacting the electrical connection material 152 to the gold braze 150 makes on oxide-resistant electrical connection. There is an insulative washer 212, which extends across the bottom of the EMI filter capacitor 132. In accordance with the present invention, there is no right-side ground metallization 142b.

Looking carefully at the right side of the capacitor in the cross-sectional view of FIG. 9, it is apparent that the right-hand edge of the capacitor 132 extends outwardly well beyond the gold braze 150. In addition to the benefits discussed above, elimination of the right-side termination has an added advantage that the EMI filter capacitor 132 can be made wider, thereby increasing the effective capacitance area, particularly, to improve its low frequency filtering characteristics. Moreover, while widening the EMI filter capacitor 132 does not make it convenient to make an electrical connection to the gold braze 150 on the right side of the feedthrough, this highlights another important aspect of the present invention. That is that one side of the EMI filter capacitor 132 no longer needs to align with the hermetic seal gold braze 150 so that the capacitor can be made wider and much more efficient in terms of its capacitance ECA and capacitance value. Widening the EMI filter capacitor 132 is desirable for increasing the capacitance value regardless of whether the terminal pins 111 are offset with respect to the center line of the ferrule 112, as shown in FIG. 9, or aligned along the center line, as shown in FIG. 7. An increased capacitance value is desirable to improve the low frequency filter performance of a filter capacitor. The term "low frequency" refers to frequencies that range from about 1 MHz to about 200 MHz, which are well below the RF pulsed frequencies of 1.5 Tesla and 3 Tesla MRI scanners.

FIG. 9A is a cross-sectional view of another embodiment of a filtered feedthrough according to the present invention, which is similar to the filtered feedthrough shown in FIG. 9, except that the ground electrode plates 146 extend to the right edge of the EMI filter capacitor 132, but the right-hand side ground metallization 142b shown in FIGs. 4, 5 and 5A is not present.

FIG. 9B is a cross-sectional view of another embodiment of a filtered feedthrough according to the present invention, which is similar to the filtered feedthrough shown in FIG. 9, except that the active electrode plates 148 extend to the right edge of the EMI filter capacitor 132, but the right-hand side ground metallization 142b shown in FIGs. 4, 5 and 5A is not present.

FIG. 9C is a cross-sectional view of another embodiment of a filtered feedthrough according to the present invention, which is similar to the filtered feedthrough shown in FIG. 9, except that both the ground and active electrode plates 146, 148 extend to the right edge of the EMI filter capacitor 132, but the right-hand side ground metallization 142b shown in FIGs. 4, 5 and 5A is not present.

FIG. 9D is a cross-sectional view of another embodiment of a filtered feedthrough according to the present invention, which is similar to the filtered feedthrough shown in FIG. 9, except that both the ground and active electrode plates 146, 148 extend to the right edge of the EMI filter capacitor 132, and the right-hand side ground metallization 142b shown in FIGs. 4, 5 and 5A is not present and has been replaced with an electrically insulative material 213.

FIG. 9E is a cross-sectional view of another embodiment of a filtered feedthrough according to the present invention, which is similar to the filtered feedthrough shown in FIG. 9, except that neither of the ground and active electrode plates 146, 148 extend to the right edge of the EMI filter capacitor 132, but the right-hand side ground metallization 142b shown in FIGs. 4, 5 and 5A is present on the right edge of the capacitor 132.

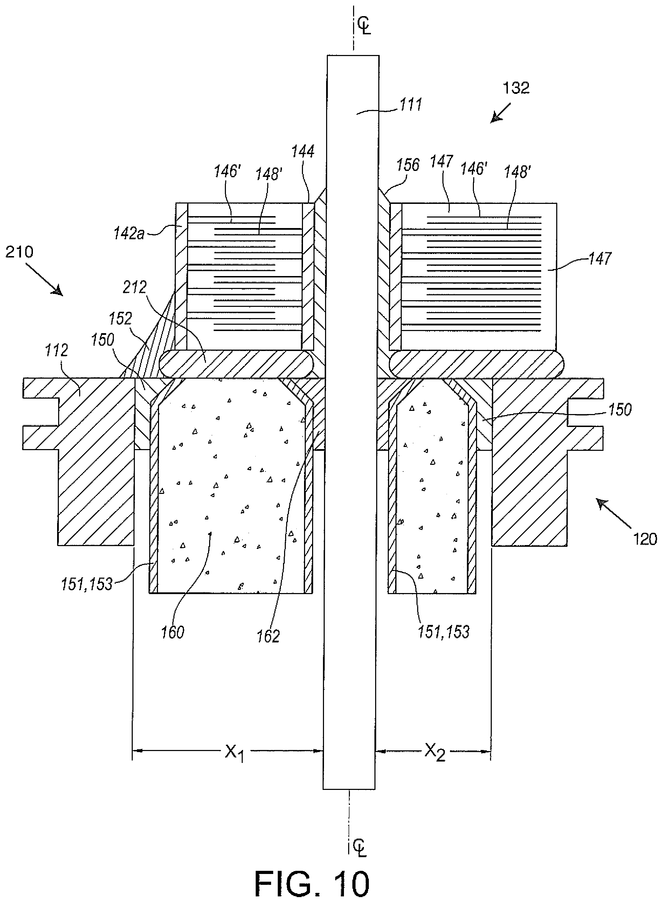

FIGURE 10 is, in a similar manner as FIG. 9, a cross-sectional view taken along line 10-10 of FIG. 8 of another embodiment of a filtered feedthrough according to the present invention where X₁ is greater than X₂. The difference between the filtered feedthroughs illustrated in FIGs. 9 and 10 is that closely-spaced pairs of active and ground electrode plates 148' and 146' are shown in the latter drawing. In a preferred embodiment, there are closely-spaced pairs of both active electrode plates 148' and ground electrode plates ground 146'. Use of closely-spaced pairs of active and ground electrode plates is very important because it greatly reduces the resistivity and inductance of the electrode plates, which completely negates elimination of the high frequency parasitic capacitance attributed to the ground metallization 142b contacted to the right-hand side of the EMI filter capacitor 132. Therefore, it is a preferred embodiment of the present invention that closely-spaced pairs electrode plates are used for both the ground electrode plates 146' and the active electrode plates 148'.

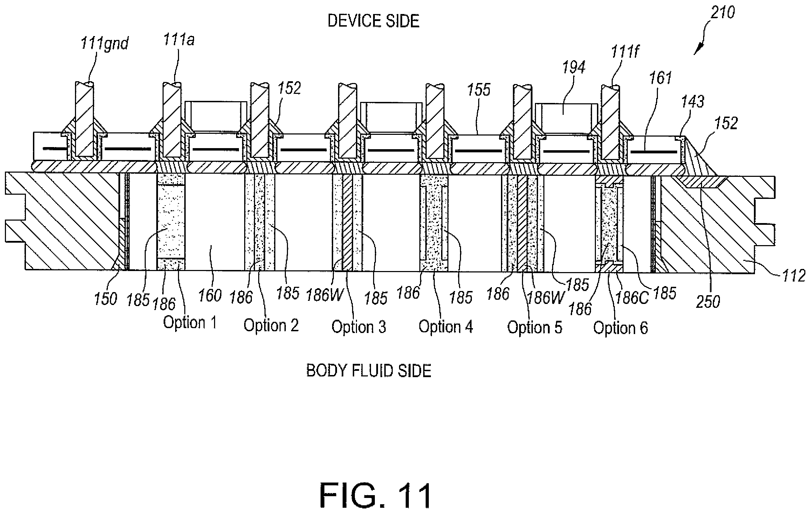

FIG. 11 illustrates an EMI filter circuit board 155 according to another embodiment the present invention with a single-sided grounded electrical connection material 152 connected to a gold braze 250. The filter circuit board may be populated with MLCC filter capacitors, X2Y attenuators of flat-thru filter capacitors, as exemplified by capacitor 194. U.S. Patent No. 11,633,612 describes mounting MLCCs, X2Y attenuators, and flat-thru capacitors to AIMD EMI filter circuit boards. The `612 patent is fully incorporated herein by reference. Numerical designation 250 refers to a gold pocket pad, which is taught by U.S. Patent Nos. 6,765,779 and 10,350,421. These patents are assigned to the assignee of the present invention and fully incorporated herein by reference.

Thus, the present invention relates to a filtered feedthrough comprising a novel EMI filter capacitor. The feedthrough comprises an insulator sealed in a ferrule opening. A terminal pin sealed in an insulator via hole has a first end that extends outwardly beyond an insulator device side. The EMI filter capacitor is positioned adjacent to the insulator device side and comprises a dielectric supporting interleaved active and ground electrode plates. A passageway extending through the dielectric has an internal metallization. An external metallization is on a terminated portion of the capacitor dielectric as opposed to an unterminated portion of the dielectric outer surface. The capacitor ground electrode plates extend to the external metallization at the terminated portion, but they do not extend to the unterminated outer surface portion. The outwardly extending terminal pin end is connected to the internal metallization in the dielectric passageway which in turn is connected to the active electrode plates. A conductive material connects the capacitor external metallization at the terminated dielectric outer surface portion to a system ground.

It is appreciated that various modifications to the inventive concepts described herein may be apparent to those skilled in the art without departing from the spirit and scope of the present invention as defined by the hereinafter appended claims.

## Claims

1. A hermetically sealed filtered feedthrough for an active implantable medical device (AIMD), the filtered feedthrough comprising:
a) an electrically conductive ferrule comprising a ferrule opening extending to a ferrule device side spaced from a ferrule body fluid side;
b) an electrically non-conductive insulator comprising an insulator outer surface extending to an insulator device side spaced from an insulator body fluid side, wherein the insulator disposed in the ferrule opening is hermetically sealed to the ferrule by a first gold braze so that when the ferrule hermetically sealed to the insulator is attached to an opening in a housing of an AIMD, the ferrule and insulator body fluid sides, and the ferrule and insulator device sides reside outside and inside the AIMD, respectively, and wherein at least one insulator via hole extends to the insulator device and body fluid sides;
c) an insulator metallization disposed on the insulator outer surface and in the insulator via hole;
d) a terminal pin residing in the insulator via hole where a second gold braze hermetically seals the terminal pin to the insulator metallization, wherein the terminal pin extends to a terminal pin first end spaced from a terminal pin second end, and wherein at least the terminal pin first end extends outwardly beyond the insulator device side;
e) a filter capacitor disposed at or adjacent to the insulator device side, the filter capacitor comprising:
i) a capacitor dielectric comprising a dielectric outer surface extending to a dielectric first major face spaced from a dielectric second major face;
ii) at least one active electrode plate and at least one ground electrode plate supported in the capacitor dielectric in an interleaved, partially overlapping capacitive relationship;
iii) a dielectric passageway extending to the dielectric first and second major faces;
iv) a capacitor internal metallization disposed in the dielectric passageway, wherein the at least one active electrode plate is connected to the capacitor internal metallization in the dielectric passageway, and wherein the outwardly extending terminal pin first end resides in the dielectric passageway where the terminal pin is conductively connected to the capacitor internal metallization connected to the at least one active electrode plate by a first conductive material, and wherein the at least one ground electrode plate is in a non-conductive relation with the capacitor internal metallization in the dielectric passageway; and
v) a capacitor external metallization disposed on a terminated outer surface portion, but not on an unterminated outer surface portion of the dielectric outer surface, wherein the at least one ground electrode plate is conductively connected to the capacitor external metallization at the terminated outer surface portion, and wherein the at least one active electrode plate is in a non-conductive relation with the capacitor external metallization; and
f) a second conductive material connecting the capacitor external metallization at the terminated dielectric outer surface portion to the ferrule or to the first braze sealing the insulator to the ferrule to provide a system ground for the filtered feedthrough.

2. The feedthrough filter of claim 1, wherein the at least one ground electrode plate does not extend to the unterminated outer surface portion of the capacitor dielectric.

3. The feedthrough filter of claim 1 or claim 2, wherein the at least one active electrode plate does not extend to the dielectric outer surface.

4. The feedthrough filter of any preceding claim, further comprising:
a) the ferrule having a rectangular shape so that in plan view looking at the ferrule device side, opposed ferrule first and second longitudinal side walls extend to and meet with opposed ferrule third and fourth curved end walls, wherein the first and second longitudinal side walls are aligned parallel to and on opposite sides of a ferrule center line that bisects the opposed third and fourth curved end walls;
b) the insulator hermetically sealed to the ferrule in the ferrule opening having opposed insulator first and second longitudinal side walls that extend to and meet with opposed insulator third and fourth curved end walls with the insulator having at least two insulator via holes residing between the insulator second longitudinal side wall and the ferrule center line; and
c) a respective one of at least two terminal pins residing in one of the at least two insulator via holes where a second gold braze hermetically seals the terminal pin to the insulator so that the at least two terminal pins reside between the insulator second longitudinal side wall and the ferrule center line, wherein each of the at least two terminal pins extends to terminal pin first and second ends with at least the terminal pin first end extending outwardly beyond the insulator device side,
preferably wherein the at least two insulator via holes are aligned parallel to the insulator second longitudinal side wall and the ferrule center line.

5. The feedthrough filter of claim 4, wherein the ferrule has an oval shape comprising the opposed ferrule first and second longitudinal side walls that extend to and meet with the opposed ferrule third and fourth curved end walls, and wherein the insulator hermetically sealed to the ferrule in the ferrule opening comprises the opposed insulator first and second longitudinal side walls that extend to and meet with the opposed insulator third and fourth curved end walls so that in plan view, the shape of the insulator matches the shape of the ferrule opening.

6. The feedthrough filter of any preceding claim, wherein, in plan view looking at the dielectric first major face, the capacitor dielectric has a rectangular shape comprising opposed dielectric first and second long sides extending to and meeting with opposed dielectric third and fourth short ends, and wherein:
the at least one ground electrode plate extends to the dielectric first long side comprising the terminated dielectric outer surface portion, but the ground electrode plate does not extend to any of the second long side and the third and fourth short ends comprising the unterminated outer surface portion of the capacitor dielectric; or
the at least one ground electrode plate extends to the first long side and to at least one of the third and fourth short ends comprising the terminated dielectric outer surface portion, but the ground electrode plate does not extend to the second long side comprising the unterminated outer surface portion of the capacitor dielectric.

7. The feedthrough filter of any preceding claim, wherein, in plan view looking at the dielectric first major face, the capacitor dielectric has a square shape comprising opposed dielectric first and second sides extending to and meeting with opposed dielectric third and fourth sides, the dielectric first and second sides being substantially equal in length to the dielectric third and fourth sides, and wherein the at least one ground electrode plate extends to the dielectric first side comprising the terminated dielectric outer surface portion, but the ground electrode plate does not extend to any of the dielectric second, third and fourth sides comprising the unterminated outer surface portion of the capacitor dielectric.

8. The feedthrough filter of any preceding claim, wherein the first gold braze is disposed at or near the ferrule device side.

9. The feedthrough filter of any preceding claim, wherein the first conductive material conductively connecting the terminal pin to the capacitor internal metallization connected to the at least one active electrode plate and the second conductive material connecting the capacitor external metallization at the terminated dielectric outer surface portion to the ferrule or to the first braze sealing the insulator to the ferrule are individually selected from the group consisting of a solder, a solder BGA, a solder paste, an epoxy, and a polyimide.

10. The feedthrough filter of any preceding claim, wherein the second conductive material connecting the capacitor external metallization to the ferrule or to the first braze sealing the insulator to the ferrule comprises a plurality of second conductive material connections at spaced intervals along the capacitor external metallization disposed on the terminated outer surface portion of the dielectric outer surface.

11. The feedthrough filter of any preceding claim, wherein the at least one active electrode plate comprises a closely-spaced pair of active electrode plates and the at least one ground electrode plate comprises a closely-spaced pair of ground electrode plates.

12. The feedthrough filter of any preceding claim, wherein the capacitor external metallization comprises an adhesion metallization that is disposed on the terminated dielectric outer surface portion and a wetting metallization that is disposed on the adhesion metallization.

13. The feedthrough filter of any preceding claim, wherein an insulative washer is disposed between the insulator and the filter capacitor.

14. The feedthrough filter of any preceding claim, wherein:
the ferrule is configured to be attachable to a housing of an active implantable medical device by a laser weld; or
the ferrule is a continuous part of an active implantable medical device housing.

15. A hermetically sealed filtered feedthrough for an active implantable medical device (AIMD), the filtered feedthrough comprising:
a) an electrically conductive ferrule comprising a ferrule opening extending to a ferrule device side spaced from a ferrule body fluid side;
b) an electrically non-conductive insulator comprising an insulator outer surface extending to an insulator device side spaced from an insulator body fluid side, wherein the insulator disposed in the ferrule opening is hermetically sealed to the ferrule by a first gold braze so that when the ferrule hermetically sealed to the insulator is attached to an opening in a housing of an AIMD, the ferrule and insulator body fluid sides, and the ferrule and insulator device sides reside outside and inside the AIMD, respectively, and wherein at least one insulator via hole extends to the insulator device and body fluid sides;
c) an insulator metallization disposed on the insulator outer surface and in the insulator via hole;
d) a terminal pin residing in the insulator via hole where a second gold braze hermetically seals the terminal pin to the insulator metallization, wherein the terminal pin extends to a terminal pin first end spaced from a terminal pin second end, and wherein at least the terminal pin first end extends outwardly beyond the insulator device side;
e) a filter capacitor disposed at or adjacent to the insulator device side, the filter capacitor comprising:
i) a capacitor dielectric comprising a dielectric outer surface extending to a dielectric first major face spaced from a dielectric second major face, wherein, in plan view looking at the dielectric first major face, the capacitor dielectric has a rectangular shape comprising opposed dielectric first and second long sides extending to and meeting with opposed dielectric third and fourth short ends;
ii) at least one active electrode plate and at least one ground electrode plate supported in the capacitor dielectric in an interleaved, partially overlapping capacitive relationship;
iii) a dielectric passageway extending to the dielectric first and second major faces;
iv) a capacitor internal metallization disposed in the dielectric passageway, wherein the at least one active electrode plate is connected to the capacitor internal metallization in the dielectric passageway, and wherein the outwardly extending terminal pin first end resides in the dielectric passageway where the terminal pin is conductively connected to the capacitor internal metallization connected to the at least one active electrode plate by a first conductive material, and wherein the at least one ground electrode plate is in a non-conductive relation with the capacitor internal metallization in the dielectric passageway; and
v) a capacitor external metallization disposed on a terminated outer surface portion, but not on an unterminated outer surface portion of the dielectric outer surface, wherein the at least one ground electrode plate extends to the dielectric first long side comprising the terminated dielectric outer surface portion, but the ground electrode plate does not extend to the second long side comprising the unterminated outer surface portion of the capacitor dielectric so that the at least one ground electrode plate is conductively connected to the capacitor external metallization at the terminated dielectric outer surface portion, and wherein the at least one active electrode plate is in a non-conductive relation with the capacitor external metallization; and
f) a second conductive material connecting the capacitor external metallization at the terminated dielectric outer surface portion to the ferrule or to the first braze sealing the insulator to the ferrule to provide a system ground for the filtered feedthrough,
preferably wherein the at least one active electrode plate does not extend to the dielectric outer surface.

16. The feedthrough filter of claim 15, wherein the at least one ground electrode plate extends to the dielectric first long side and to at least one of the dielectric third and fourth short sides comprising the terminated dielectric outer surface portion, but the ground electrode plate does not extend to the second long side comprising the unterminated outer surface portion of the capacitor dielectric so that the at least one ground electrode plate is conductively connected to the capacitor external metallization at the terminated dielectric outer surface portion.

17. The feedthrough filter of claim 15 or claim 16,
a) wherein the ferrule has a rectangular shape comprising opposed ferrule first and second longitudinal side walls that extend to and meet with opposed ferrule third and fourth curved end walls, and
b) wherein the ferrule first and second longitudinal side walls are aligned parallel to and on opposite sides of a ferrule center line that bisects the opposed ferrule third and fourth curved end walls, and
c) wherein the insulator hermetically sealed to the ferrule in the ferrule opening has opposed insulator first and second longitudinal side walls that extend to and meet with opposed insulator third and fourth curved end walls with the insulator having at least two insulator via holes residing between the insulator second longitudinal side wall and the ferrule center line, and
d) wherein a respective one of at least two terminal pins reside in one of the at least two insulator via holes where a second gold braze hermetically seals the terminal pin to the insulator, and
e) wherein each of the at least two terminal pins extend to terminal pin first and second ends with at least the terminal pin first end extending outwardly beyond the insulator device side, and
f) wherein the capacitor dielectric has at least two dielectric passageways extending through the dielectric first major face with a capacitor internal metallization disposed in each of the plurality of dielectric passageways, and
g) wherein the at least one active electrode plate is connected to the capacitor internal metallization in each of the at least two dielectric passageways, and
h) wherein a respective one of the at least two outwardly extending terminal pin first ends reside in one of the at least two dielectric passageways with the first conductive material conductively connecting the terminal pin to the capacitor internal metallization connected to the at least one active electrode plate.

18. The filtered feedthrough of any of claims 15 to 17, wherein:
a) the ferrule has a rectangular shape comprising opposed ferrule first and second longitudinal side walls that extend to and meet with opposed ferrule third and fourth curved end walls;
b) the insulator disposed in the ferrule opening comprises opposed insulator first and second longitudinal sides that extend to and meet with opposed insulator third and fourth curved ends so that the shape of the insulator matches the shape of the ferrule opening with the first gold braze hermetically sealing the insulator to the ferrule;
c) the filter capacitor has a rectangular shape so that in plan view looking at the dielectric first major face, opposed dielectric first and second long sides extend to and meet with opposed dielectric third and fourth short ends;
d) the at least one ground electrode plate is conductively connected to the capacitor external metallization disposed on the dielectric first long side as a terminated outer surface portion, but the ground electrode plate does not extend to the dielectric second long side as an unterminated outer surface portion of the dielectric outer surface, and wherein the at least one active electrode plate does not extend to the capacitor external metallization at the dielectric outer surface,
e) wherein the dielectric first long side resides spaced above the first gold braze hermetically sealing the insulator to the ferrule, and the dielectric second long side extends laterally outwardly beyond the first gold braze; and
f) the second conductive material conductively connects the capacitor external metallization at the dielectric first long side serving as the terminated outer surface portion to the ferrule or to the first braze sealing the insulator to the ferrule to provide a system ground for the filtered feedthrough.

19. A hermetically sealed filtered feedthrough for an active implantable medical device (AIMD), the filtered feedthrough comprising:
a) an electrically conductive ferrule comprising a ferrule opening extending to a ferrule device side spaced from a ferrule body fluid side;
b) an electrically non-conductive insulator comprising an insulator outer surface extending to an insulator device side spaced from an insulator body fluid side, wherein the insulator disposed in the ferrule opening is hermetically sealed to the ferrule by a first gold braze so that when the ferrule hermetically sealed to the insulator is attached to an opening in a housing of an AIMD, the ferrule and insulator body fluid sides, and the ferrule and insulator device sides reside outside and inside the AIMD, respectively, and wherein at least one insulator via hole extends to the insulator device and body fluid sides;
c) an insulator metallization disposed on the insulator outer surface and in the insulator via hole;
d) a terminal pin residing in the insulator via hole where a second gold braze hermetically seals the terminal pin to the insulator metallization, wherein the terminal pin extends to a terminal pin first end spaced from a terminal pin second end, and wherein at least the terminal pin first end extends outwardly beyond the insulator device side;
e) a filter capacitor disposed at or adjacent to the insulator device side, the filter capacitor comprising:
i) a capacitor dielectric comprising a dielectric outer surface extending to a dielectric first major face spaced from a dielectric second major face;
ii) at least one active electrode plate and at least one ground electrode plate supported in the capacitor dielectric in an interleaved, partially overlapping capacitive relationship;
iii) a dielectric passageway extending to the dielectric first and second major faces;
iv) a capacitor internal metallization disposed in the dielectric passageway, wherein the at least one active electrode plate is connected to the capacitor internal metallization in the dielectric passageway, and wherein the outwardly extending terminal pin first end resides in the dielectric passageway where the terminal pin is conductively connected to the capacitor internal metallization connected to the at least one active electrode plate by a first conductive material, and wherein the at least one ground electrode plate is in a non-conductive relation with the capacitor internal metallization in the dielectric passageway; and
v) a capacitor external metallization disposed on at least a portion of the dielectric outer surface to provide a terminated dielectric outer surface portion, wherein the at least one ground electrode plate extends to a first segment of the terminated dielectric outer surface portion, but the at least one ground electrode plate does not extend to a second segment of the terminated dielectric outer surface portion, and wherein the at least one active electrode plate is in a non-conductive relation with the capacitor external metallization; and
f) a second conductive material connecting the first portion of the capacitor external metallization on the dielectric outer surface portion to the ferrule or to the first braze sealing the insulator to the ferrule to provide a system ground for the filtered feedthrough.

20. The filtered feedthrough of claim 19, wherein, in plan view looking at the dielectric first major face, the capacitor dielectric has a rectangular shape comprising opposed dielectric first and second long sides extending to and meeting with opposed dielectric third and fourth short ends, and capacitor the external metallization is disposed on both of the dielectric first and second long sides comprising the terminated dielectric outer surface portion, and wherein the at least one ground electrode plate extends to the first long side of the capacitor dielectric as the first segment of the terminated dielectric outer surface portion, but the at least one ground electrode plate does not extend to the second long side of the capacitor dielectric as the second segment of the terminated dielectric outer surface portion, and wherein the at least one active electrode plate is in a non-conductive relation with the capacitor external metallization, and
preferably wherein the dielectric third and fourth short ends are also provided with the external metallization and the at least one ground electrode plate either does or does not extend to the third and fourth short ends of the capacitor dielectric outer surface.
